# EUROPEAN PATENT APPLICATION

(11) **EP 3 494 949 A1**
(43) Date of publication of application: **12.06.2019**
(21) Application number: 18160258.2
(22) Date of filing: 06.03.2018
(51) Int. Cl.: A61H 39/06

(54) **INTELLIGENT MOXYBUSTION DEVICE**

(30) Priority: 06.12.2017 CN 201711279710; 06.12.2017 CN 201721681946 U
(71) Applicant: Li Shizhen (Guangzhou) Health Technology Co., Ltd., 511400 Guangzhou City, Guangdong (CN)
(72) Inventor: LIU, Xiaojun, Guangzhou city, Guangdong 511400 (CN)
(74) Representative: ZHAOffice SPRL

(57) **Abstract**

The invention provides an intelligent moxibustion device, comprising a vertically disposed barrel (1), an ash tower (2) buckled with the barrel, a moxa stick driving unit and an intelligent control unit disposed in the barrel; wherein the moxa stick driving unit comprises a moxa stick sleeve (3) and a stepping motor (4) disposed above the moxa stick sleeve; the stepping motor drives the moxa stick to move up and down through the transmission of a gear (41); the intelligent control unit comprises a battery (6) disposed above the stepping motor for powering the entire moxibustion device, a circuit board (7) fixed above the battery, and a temperature sensor disposed in the ash tower, the battery being connected to the circuit board to provide power to the circuit board, the circuit board being connected to the temperature sensor through input and output terminals. The present invention uses the temperature sensor to sense the progress of the burning of the moxa stick, when the temperature is lower than a rated value, the sensor sends a signal to the circuit board, the circuit board controls the stepping motor to move the moxa stick downward through a mechanical transmission, thereby ensuring a constant temperature.

## Description

### Technical Field

The present invention relates to the technical field of moxibustion devices, and in particular, to an intelligent moxibustion device, which realizes intelligent operations through a control system and an automatic transmission, without manual operations in the whole treatment process.

### Background

Moxibustion is a traditional Chinese medical treatment method that uses a burning moxa stick to perform a suspended moxibustion on human acupoints. Moxibustion originated in the ancient Chinese era, and is a treatment method that utilizes moxibustion-heat generated by moxibustion materials made of moxa to stimulate acupoints on the body surface or specific parts of the body, adjusts disordered human physiological and biochemical functions by stimulating the activity of qi ( 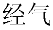 ), so as to achieve the purpose of disease prevention and treatment.

With the development of moxibustion therapy, moxibustion devices have emerged. A moxibustion device can be fixed on any part of the limbs, and the person who is experiencing moxibustion can adjust the distance between moxa grains and the skin by him or herself. When moxa grains (moxa stick) are burned in a relatively sealed moxibustion device, the heat generated is not easily dissipated and has a long lasting effect on acupoints. The device has the advantages of firepower balance, strong penetration, and uninterrupted function. The device makes full use of beneficial effects generated in the process of burning moxa leaves in the treatment, such as thermal effect, medical effect and smoking effect, to stimulate acupoints and meridians. The device combines the thermal effect, the medical effect and the smoking effect together, improving the treatment effects of conventional moxibustion. Currently, the well known structure of a moxibustion device is combined by a moxa stick, a paper shell, a cap with a pin or an upper cover formed by magnet and a paper shell, and a double-sided adhesive attached to the hand. The moxa stick is lit manually through an open flame and then inserted onto the pin of the upper cover, the upper cover is then inserted into the box body, which is adhered to the skin through the adhesive, and then moxibustion works. However, the product has several major problems: first, the open flame is easy to cause danger; second, with the continuous burning of the moxa stick, the temperature felt by the skin at the contact gradually decreases, and at this time, the depth of the moxa stick inserted in the product needs to be adjusted manually, which is impossible to quantify, leading to uneven effect; third, the used paper shell might cause a fire during the high-temperature moxibustion with the moxa stick; and fourth, ashes generated during the combustion process of the moxa stick might easily scald the skin when falling to the skin.

### Summary

In view of the shortcomings of the prior art, the object of the present invention is to provide an intelligent moxibustion device, to solve the problems raised in the background art hereinabove. The invention realizes intelligent operations through a control system and an automatic transmission, without manual operations in the whole treatment process.

In order to solve the abovementioned problems, the present invention provides an intelligent moxibustion device, comprising a vertically disposed barrel, an ash tower buckled with the barrel, a moxa stick driving unit and an intelligent control unit disposed in the barrel. The moxa stick driving unit comprises a moxa stick sleeve and a stepping motor disposed above the moxa stick sleeve. The stepping motor drives, through the transmission of a gear, the moxa stick to move up and down. The intelligent control unit comprises a battery disposed above the stepping motor for powering the entire moxibustion device, a circuit board fixed above the battery, and a temperature sensor disposed in the ash tower. The battery is connected to the circuit board to provide power to the circuit board, and the circuit board is connected to the temperature sensor through input and output terminals for receiving and analyzing a temperature signal below the moxibustion device during moxibustion.

Further, the ash tower comprises a fine mesh screen disposed at the center of the tower, strip support tables uniformly disposed on the upper surface of the fine mesh screen, and a resistance heating wire disposed on the strip support tables, wherein the resistance heating wire is disposed directly below the moxa stick, and the ash tower is provided, on the outer side wall thereof, with air circulation holes for ensuring the full combustion of the moxa stick.

Further, the fine mesh screen is provided, on the outer edges thereof, with threaded holes, via which the temperature sensor is fixed within the ash tower.

Further, the output shaft of the stepping motor is connected to a rack and gear mechanism, the rack of the rack mechanism is mounted in a motor fixing seat that limits the degree of freedom of the rack, and the rack is provided, on both ends of the range thereof, with stroke switches that limit the position of the rack.

Further, the moxa stick sleeve includes a fixing barrel fixed to the bottom of the motor fixing seat, a moxa stick seat that is sleeved in the fixing barrel and is capable of moving up and down, and channels reserved on the side walls of the fixing barrel for the moxa stick seat to move up and down. The side walls of the moxa stick seat is provided with connecting ears. The moxa stick seat is connected to the lower end of the rack through the connecting ears passing through the channels on the side walls of the fixing barrel. The stepping motor drives the rack to move up and down, through the rotation of the output shaft, such that the moxa stick seat causes the moxa stick to move up and down.

Further, the barrel is provided, at the top thereof, with a power button for turning on and turning off the whole moxibustion device.

Further, the circuit board includes a main control circuit, a stroke switch circuit, an ignition circuit, a temperature detection circuit, a one-button switch circuit, a button state detection circuit, a charging circuit, and a motor forward and reverse rotation circuit. The main control circuit is connected to the stroke switch circuit, the ignition circuit, the temperature detection circuit, the one-button switch circuit, the button state detection circuit, the motor forward and reverse rotation circuit through input and output interfaces. The input terminal of the charging circuit is connected to an external power source, the output terminal of the charging circuit is connected to the one-button switch circuit and the button state detection circuit for supplying power to the device, and the output terminal of the charging circuit is connected to the battery at the same time for charging the battery. The input terminal of the stroke switch circuit is externally connected to the stroke switch for limiting the up and down positions of the rack to prevent the rack from being detached from the gear. The input terminal of the temperature detection circuit is connected to the temperature sensor to receive the temperature signal measured by the temperature sensor. The motor forward and reverse rotation circuit is connected to the stepping motor to control the forward and reverse rotation of the stepping motor. The ignition circuit is connected to the resistance heating wire for igniting the moxa stick. The one-button switch circuit and the button state detection circuit are connected to the power button through the input terminal, such that a long-time press achieves ignition and a short-time press turns on/off the device.

More specifically, the main control circuit adopts STM8S003 main chip and comprises twenty input and output ports, wherein the PD3 port is connected to the temperature detection circuit, the PD2 port is connected to the ignition circuit, the PB4 port and the PB5 port are connected to the stroke switch circuit.

The charging circuit is connected to the one-button switch circuit and the button state detection circuit. The LED indicator in the charging circuit displays two colors, blue color and red color, so as to display the state of charge of the battery. When the battery is fully charged, blue color is displayed, and when the power is less than 10%, red color is displayed.

The PC6 and PC7 ports of the main chip are connected to the one-button switch circuit and the button state detection circuit, and also a short-time press turns on/off the device and a long-time press achieves ignition. The PC3 port and the PC4 port of the main chip are connected to the motor forward and reverse rotation circuit to control the forward and reverse rotation of the motor so as to achieve the rise and fall of the moxa stick.

More specifically, the barrel is made of bakelite material, which is resistant to high temperature and avoids the danger of fire due to high temperature. The periphery of the resistance heating wire is insulated by ceramic to avoid the danger of scalding the user.

The working principle and beneficial effects of the present invention are as follows: first, the moxa stick heating process of the invention is carried out inside the product, the moxa stick is placed completely into the product to burn inside by being heated by the resistance wire, no open flame can be seen, and the periphery of the resistance heating wire is insulated by ceramic.

Second, the present invention uses a temperature sensor to sense the progress of the burning of the moxa stick, when the temperature is lower than a rated value, the sensor sends a signal to the circuit board, the circuit board controls the stepping motor to move the moxa stick downward through a mechanical transmission, thereby ensuring a constant temperature.

Third, the outer shell of the product of the present invention is made of bakelite material, which is a high-temperature resistant plasticity phenolic plastic and is unlikely to cause a fire.

Fourth, the double-layer ash tower designed according to the present invention ensures that after the moxa stick is burned, ash will not fall to the skin. The first layer is an electric wire; the second layer is an ash-accommodation tower, and the electric wire and the ash-accommodation are dislocated of the holes of the electric heating wire. Neither ash falls to the skin nor heat conduction is affected.

Fifth, in terms of user experience, in the present invention, all work can be done automatically by pressing the button switch on the top once, freeing the two hands, such that moxibustion can be performed in a more intelligent and appropriate manner, solving the issues during use involved with fire risk and complex usage processes.

### Brief Description of the Drawings

Fig. 1 is a schematic exploded structure of the present invention;
Fig. 2 is a schematic partial exploded structure of the present invention;
Fig. 3 is a schematic structural diagram of an ash tower of the present invention comprising a resistance heating wire;
Fig. 4 is a schematic structural diagram of a moxa stick seat of the present invention;
Fig. 5 is a diagram showing the principle of the structure of an intelligent control unit of the present invention;
Fig. 6 is a structural diagram of a main control circuit of the present invention;
Fig. 7 is a structural diagram of a stroke switch circuit of the present invention;
Fig. 8 is a structural diagram of an ignition circuit of the present invention;
Fig. 9 is a structural diagram of a temperature detection circuit of the present invention;
Fig. 10 is a structural diagram of a charging circuit of the present invention;
Fig. 11 is a diagram of a one-button switch structure of the present invention;
Fig. 12 is a diagram of a motor forward and reverse rotation structure of the present invention.

Reference signs: 1 for barrel, 2 for ash tower, 21 for fine mesh screen, 22 for strip support tables, 23 for resistance heating wire, 24 for protrusion, 25 for ceramic thermally insulating region, 26 for air circulation holes, 3 for the moxa stick sleeve, 31 for fixing barrel, 32 for moxa stick seat, 33 for fixing pin, 34 for connecting ears, 35 for channels, 4 for stepping motor, 41 for gear, 42 for rack, 43 for connecting shaft, 5 for motor fixing seat, 6 for battery, 7 for circuit board, 78 for stroke switch, 8 for LED indicator, 9 for power button, 10 for moxa stick, 11 for battery fixing seat, 12 for transparent aperture, 13 for hinge member, 14 for threaded seat, 15 for detaching button.

### Detailed Description of the Embodiments

In order to make the technical means, the creative features, the achievement of the object and the effect of the present invention easy to understand, the present invention will be further described with reference to the specific embodiments. It should be understood that the specific embodiments described herein are merely for explaining the present invention, rather than limiting the present invention.

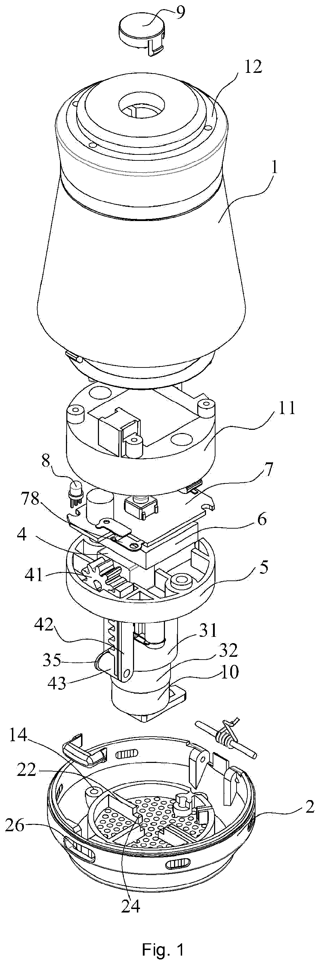

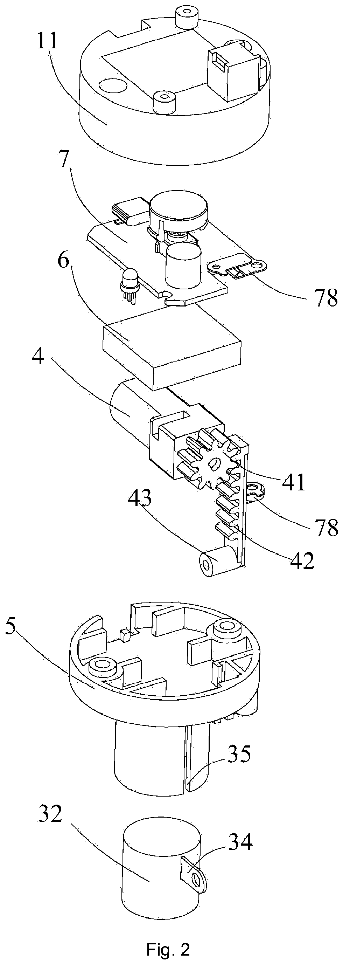

With reference to Figs. 1 and 2, provided in an embodiment of the present invention is an intelligent moxibustion device, which intelligently keeps the moxa stick to be burned at an effective distance of 10-15mm continuously to maximize the effect of moxibustion, solving the problem that the moxibustion stick gets away from the skin farther and farther when being burned in the moxibustion device on the market, and can not achieve the best moxibustion effect. No open flame is used for combustion in the embodiment, which mainly includes: an intelligent moxibustion device, comprising a vertically disposed barrel 1, an ash tower 2 which is buckled with the barrel 1, an moxa stick driving unit and an intelligent control unit which are disposed in the barrel 1.

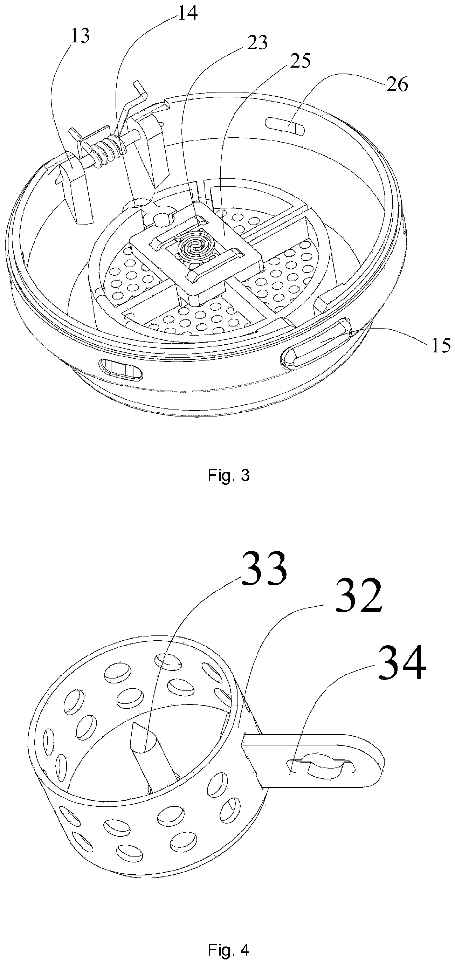

As shown in Fig. 3, the ash tower 2 includes a fine mesh screen 21 disposed at the center of the tower, strip support tables 22 uniformly disposed on the upper surface of the fine mesh screen 21, a resistance heating wire 23 disposed on the strip support tables 22. The resistance heating wire 23 is disposed directly below the moxa stick. The fine mesh screen 21 has a circular structure. There are three strip support tables 22 along the diameter of the fine mesh screen 21. Each strip support table 22 is provided with a protrusion 24 close to the center of the circle. The resistance heating wire 23 is fixed on the protrusion 24, and the upper surface of the resistance heating wire 23 is flat with the upper surface of the strip support bar.

A ceramic thermally insulating tape 25 is provided around the resistance heating wire 23 to prevent the user from being scalded.

The outer wall of the ash tower 2 is provided with air circulation holes 26 for ensuring sufficient combustion of the moxa stick.

As shown in Fig. 2, the moxa driving unit includes a moxa stick sleeve 3 and a stepping motor 4 disposed above the moxa stick sleeve 3. The stepping motor 4 drives the moxa stick to move up and down through the transmission of a gear. The moxa stick sleeve includes: a fixing barrel 31, and a moxa stick seat 32 sleeved in the fixing barrel 31 and being capable of moving up and down. A fixing pin 33 is vertically disposed in the moxa stick seat, and the moxa stick can be inserted onto the fixing pin 33. The moxa stick seat is provided, on the side walls thereof, with connecting ears 34. Channels 35 are reserved on the side walls of the fixing barrel 31 for the moxa stick seat to move up and down. The stepping motor 4 is provided, at the output shaft end thereof, with a gear 41, and a rack 42 engaged with the gear 41. The rack 42 is provided, at the lower end thereof, with a connecting shaft 43. The connecting shaft 43 is fixedly connected to the connecting ears 34 that pass through the channels 35 on the side walls of the fixing barrel 31. The stepping motor 4 drives the rack 42 to move up and down through the rotation of the output shaft, so that the moxa stick seat 32 causes the moxa stick to move up and down. The one-button switch circuit and the button state detection circuit are connected to the power button through the output terminal, such that a long-time press achieves ignition and a short-time press turns on/off the device.

As shown in Fig. 4, more specifically, the side surface of the moxa stick seat 32 is a hollow cylindrical structure, and the bottom surface of the moxa stick seat 32 is provided with a fixing pin.

As a preferred solution of the present invention, the rack of the rack mechanism of the stepping motor is mounted in a motor fixing seat 5 that limits the degree of freedom (mobility?) of the rack. The rack 42 is provided, at both ends of the range thereof, with stroke switches 78 for limiting the position of the rack.

As shown in Fig. 2, the intelligent control unit includes a battery 6 above the stepping motor 4 for powering the entire moxibustion device, a circuit board 7 fixed above the battery 6, and a temperature sensor disposed in the ash tower 2, the battery 6 being connected to the circuit board 7 to provide power to the circuit board 7, the circuit board 7 being connected to the temperature sensor through the input and output terminals for receiving and analyzing the temperature signal below the moxibustion device during moxibustion.

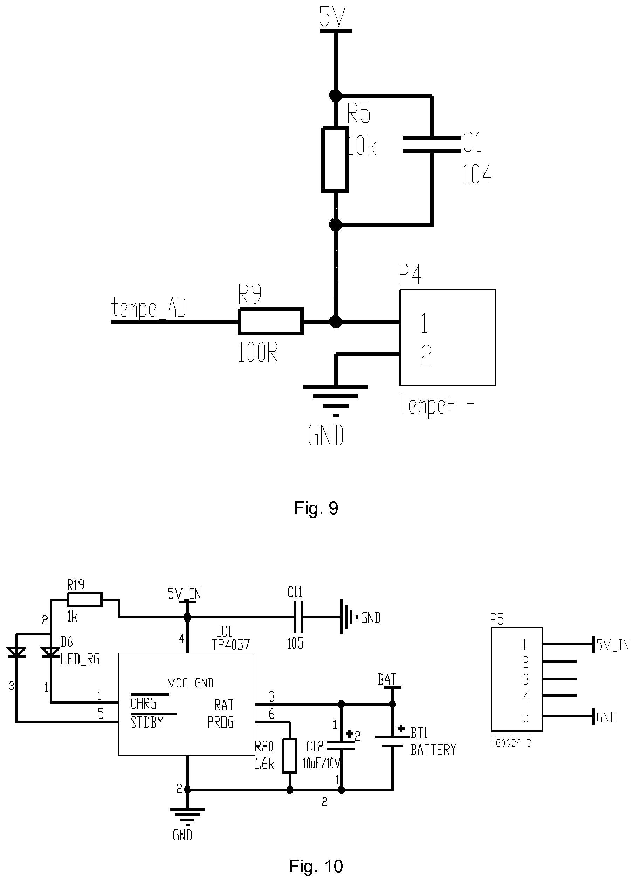

As shown in Fig. 1 and Fig. 10, as a preferred solution of the present invention, the battery 7 is fixed in the battery fixing seat 11. The circuit board 7 is fixed above the battery fixing seat 11, and is provided with, on one side thereof, with an LED indicator 8 for displaying the battery level. and is provided with, at a position where the barrel is close to the power button 9, with a transparent aperture 12. The LED indicator 8 emits light to the transparent aperture 12, and the LED indicator 8 includes a blue light circuit and a red light circuit connected in parallel, with an "Or" switch disposed between the blue light circuit and the red light circuit, so as to turn on the blue light circuit when the power is full and turn on the red light circuit when the power is less than 10%.

More specifically, provided on the barrel 1 and below the transparent aperture 12 is a USB charging interface (not shown) for charging the battery 7.

Wherein, the motor fixing seat 5 and the battery fixing seat 11 can be buckled together, which not only saves space but also stabilizes the internal structure.

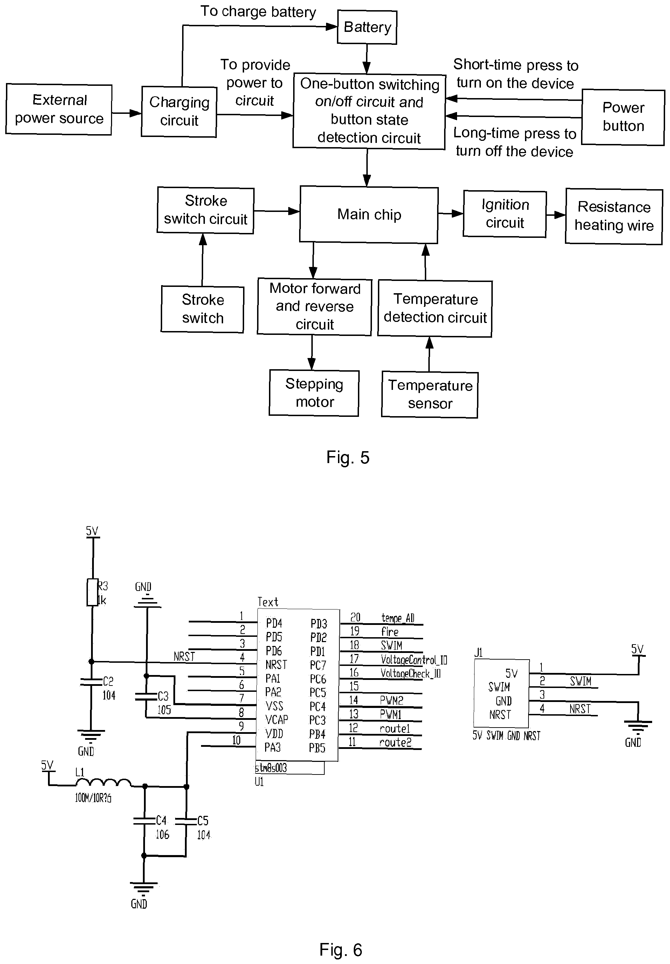

As shown in Figs. 3 and 5, the circuit board includes a main control circuit, a stroke switch circuit, an ignition circuit, a temperature detection circuit, a one-button switch circuit and a button state detection circuit, a charging circuit, and a motor forward and reverse rotation circuit. The main control circuit is connected to the stroke switch circuit, the ignition circuit, the temperature detection circuit, the one-button switch circuit, the button state detection circuit, the motor forward and reverse rotation circuit through input and output interfaces. The input terminal of the charging circuit is connected to an external power source, the output terminal of the charging circuit is connected to the one-button switch circuit and the button state detection circuit for supplying power to the device, and the output terminal of the charging circuit is connected to the battery at the same time for charging the battery. The input terminal of the stroke switch circuit is externally connected to the stroke switch for limiting the up and down positions of the rack to prevent the rack 42 from being detached from the gear.

The input terminal of the temperature detection circuit is connected to the temperature sensor to receive the temperature signal measured by the temperature sensor. The temperature sensor adopts a thermistor that is sensitive to temperature measurement and has a long lifetime.

The temperature sensor is fixed onto the ash tower through the threaded seat on the ash tower.

The motor forward and reverse rotation circuit is connected to the stepping motor 4 to control the forward and reverse rotation of the stepping motor 4. The ignition circuit is connected to the resistance heating wire 23 for igniting the moxa stick. The one-button switch circuit and the button state detection circuit are connected to the power button 9 through the input terminal, such that a long-time press achieves ignition and a short-time press turns on/off the device.

As shown in Fig. 6, more specifically in the present invention, the main control circuit adopts STM8S003 main chip and comprises twenty input and output ports, and the main control circuit mainly receives and processes signals and sends control signals.

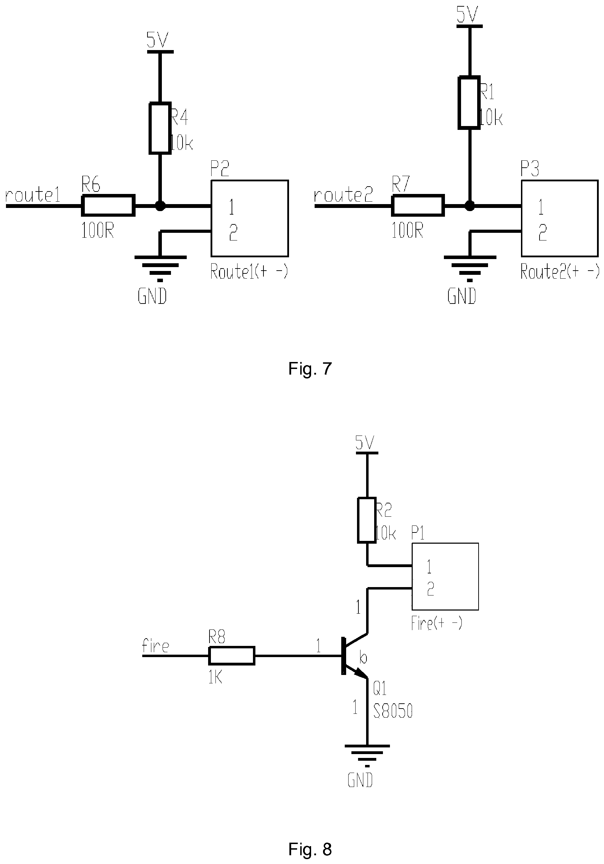

As shown in Fig. 7, the stroke switch circuit includes two stroke switches. When the two contacts of one of the stroke switches contact, the main chip receives a closing signal from the stroke switch via PB4 (or PB5). When the rack has completed the stroke, the main chip controls the motor via PC4 and PC5 at the same time, and generates correspondly a motor stop signal. By providing the PC4 and PC5 pins with different levels, the main chip can achieve the forward and reverse rotation and stop of the motor.

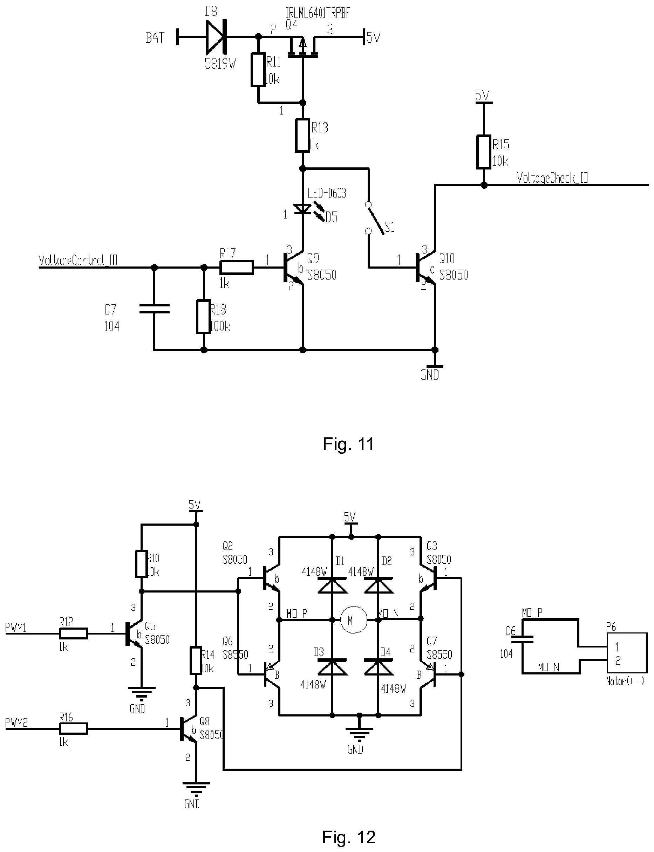

As shown in Fig. 8 and Fig. 11, the main chip is connected to the one-button switch circuit and the button state detection circuit via the voltage detection port PC6 and the voltage control port PC7. The main chip is connected to the ignition circuit via the PD2 port. When the device is turned on, the main control circuit determines whether there is a button S1 to be pressed by detecting the level change at PC6. When the PC6 level is low, it is determined there is a button being pressed, the corresponding processing is performed at the time. When it is determined that there is a short-time press, the processor controls the PC7 pin to output a low level to turn off the power switch, thereby turning off the device. When it is determined that there is a long-time press, the processor controls the PD2 pin to output a high level to turn on the ignition circuit, thereby achieving the ignition function, that is, a long-time press achieves ignition.

When the device is to be turned on, a short-time press achieves the turning on, and after being powered on, the main chip controls the PC7 pin to output a high electric level, the power button is self-locked, so as to lock the power path, achieving the turning on.

As shown in Fig. 9 and Fig. 12, the main chip is connected to the temperature detection circuit via the PD3 port, the PC3 port and the PC4 port of the main chip are connected to the forward and reverse rotation circuit of the motor to control the forward and reverse rotation of the motor, thereby achieving the rise and fall of the moxa stick. Temperature information is collected via PD3 so as to know the temperature changes. When the temperature is too high, the motor is controlled to rotate reversely to let the distance between the moxa stick and the skin surface increase, so that the temperature drops to the temperature of the skin and keeps stable, vice versa.

As shown in Fig. 10, the charging circuit includes a power chip TP4057. The charging circuit is connected to the one-button switch circuit and the button state detection circuit via a BAT output terminal. The LED indicator in the charging circuit displays two colors, blue color and red color, thereby showing the state of charge of the battery. When the battery is fully charged, blue color is displayed, and when the power is less than 10%, red color is displayed.

As a preferred solution of the present invention, one side of the connection between the ash tower 2 and the upper barrel 1 is hingedly connected by a hinge member 13, and the other side is connected by a buckle, with a detaching button 15 being provided at the buckling portion. This connection manner can prevent the loss of the ash tower, and is more convenient and quick to use, compared to the threaded connection.

The working principle of the invention is as follows: open the ash tower by means of the detaching button, place the moxa stick on the moxa stick seat, clasp the ash tower, fixing the moxibustion device on the moxibusted part through the double-sided adhesive, press the top power button, the circuit board starts the program to run, the resistance wire heats the moxa stick. As the moxa stick is gradually burned, when the temperature is lower than the temperature of the temperature sensor, the temperature sensor sends a signal to the circuit board, and the circuit board drives the stepping motor to drive the moxa stick drop through the mechanical transmission of the gear to reach a constant temperature.

The foregoing is only about the preferred embodiments of the present invention, and the structure there is not limited to the above-mentioned shape. Any modifications, equivalent substitutions and improvements made within the spirit and principle of the present invention should be encompassed in the scope of protection of the present invention.

## Claims

1. An intelligent moxibustion device, comprising a vertically disposed barrel and an ash tower buckled with the barrel, **characterized by** further comprising a moxa stick driving unit and an intelligent control unit disposed in the barrel; wherein the moxa stick driving unit comprises a moxa stick sleeve and a stepping motor disposed above the moxa stick sleeve; the stepping motor drives the moxa stick to move up and down through the transmission of a gear; the intelligent control unit comprises a battery disposed above the stepping motor for powering the entire moxibustion device, a circuit board fixed above the battery, and a temperature sensor disposed in the ash tower, the battery being connected to the circuit board to provide power to the circuit board, the circuit board being connected to the temperature sensor through input and output terminals for receiving and analyzing a temperature signal below the moxibustion device during moxibustion.

2. The intelligent moxibustion device according to claim 1, **characterized in that**, the ash tower comprises a fine mesh screen disposed at the center of the tower, strip support tables uniformly disposed on the upper surface of the fine mesh screen, and a resistance heating wire disposed on the strip support tables; wherein the resistance heating wire is disposed directly below the moxa stick; the ash tower is provided, on the outer side wall thereof, with air circulation holes for ensuring the full combustion of the moxa stick.

3. The intelligent moxibustion device according to claim 2, **characterized in that**, the output shaft of the stepping motor is connected to a rack and gear mechanism, the rack of the rack mechanism is mounted in a motor fixing seat that limits the degree of freedom of the rack, and the rack is provided, on both ends of the range thereof, with stroke switches that limit the position of the rack.

4. The intelligent moxibustion device according to claim 3, **characterized in that**, the moxa stick sleeve includes a fixing barrel fixed to the bottom of the motor fixing seat, a moxa stick seat that is sleeved in the fixing barrel and can move up and down, and channels reserved on the side walls of the fixing barrel for the moxa stick seat to move up and down, the side walls of the moxa stick seat being provided with connecting ears, the moxa stick seat being connected to the lower end of the rack through the connecting ears passing through the channels on the side walls of the fixing barrel, the stepping motor driving the rack to move up and down through the rotation of the output shaft such that the moxa stick seat moves the moxa stick up and down.

5. The intelligent moxibustion device according to claim 4, **characterized in that**, the top of the barrel is provided with a power button for turning on and turning off the whole moxibustion device.

6. The intelligent moxibustion device according to claim 5, **characterized in that**, the circuit board includes a main control circuit, a stroke switch circuit, an ignition circuit, a temperature detection circuit, a one-button switch circuit and a button state detection circuit, a charging circuit, and a motor forward and reverse rotation circuit; wherein, the main control circuit is connected to the stroke switch circuit, the ignition circuit, the temperature detection circuit, the one-button switch circuit, the button state detection circuit, the motor forward and reverse rotation circuit through input and output interfaces; the input terminal of the charging circuit is connected to an external power source, the output terminal of the charging circuit is connected to the one-button switch circuit and the button state detection circuit for supplying power to the device, and the output terminal of the charging circuit is connected to the battery at the same time for charging the battery; the input terminal of the stroke switch circuit is externally connected to the stroke switch for limiting the up and down positions of the rack to prevent the rack from being detached from the gear; the input terminal of the temperature detection circuit is connected to a temperature sensor to receive a temperature signal measured by the temperature sensor; the motor forward and reverse rotation circuit is connected to the stepping motor to control the forward and reverse rotation of the stepping motor; the ignition circuit is connected to the resistance heating wire for igniting the moxa stick; the one-button switch circuit and the button state detection circuit are connected to the power button through the input terminal, such that a long-time press achieves ignition and a short-time press turns on/off the device.

7. The intelligent moxibustion device according to claim 1, **characterized in that**, the fine mesh screen is provided, on the outer edges thereof, with a threaded seat via which the temperature sensor is fixed within the ash tower.

8. The intelligent moxibustion device according to claim 7, **characterized in that**, the circuit board is provided with, on one side thereof, with an LED indicator for displaying the battery level, and is provided with, at a position where the barrel is close to the power button, with a transparent aperture; the LED indicator includes a blue light circuit and a red light circuit connected in parallel, with an "Or" switch disposed between the blue light circuit and the red light circuit, so as to turn on the blue light circuit when the power is full and turn on the red light circuit when the power is less than 10%.

9. The intelligent moxibustion device according to claim 1, **characterized in that**, one side of the connection between the ash tower and the upper barrel is hingedly connected by a hinge member, and the other side is connected by a buckle, with a detaching button being provided at the buckling portion.

10. The intelligent moxibustion device according to any one of claims 1 to 9, **characterized in that**, the barrel is made of bakelite material, and a ceramic thermally insulating region is used around the resistance heating wire.
